# EUROPEAN PATENT APPLICATION

(11) **EP 3 656 381 A1**
(43) Date of publication of application: **27.05.2020**
(21) Application number: 18208109.1
(22) Date of filing: 23.11.2018
(51) Int. Cl.: A61K 31/145, A61P 11/12

(54) **ANALOGUES OF CYSTEAMINE AS THERAPEUTIC AGENTS FOR CYSTIC FIBROSIS**

(71) Applicant: I.E.R.F.C. European Institute for Cystic Fibrosis Research, 20132 Milano (IT)
(72) Inventor: MAIURI, Luigi, "deceased" (IT); VILLELLA, Valeria, 20132 Milano (IT); BORELLA, Fabio, 20132 Milano (IT); COZZA, Giorgio, 20132 Milano (IT); VENERANDO, Andrea, 20132 Milano (IT)
(74) Representative: Bianchetti Bracco Minoja S.r.l.

(57) **Abstract**

The present invention concerns compounds of formula I 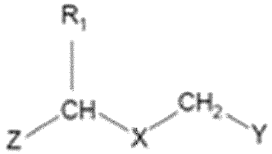 Wherein X. Y and Z are as defined in the description, for use in the treatment of cystic fibrosis.

## Description

The present invention concerns analogues of cysteamine of formula I 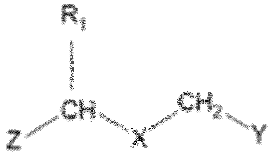 for use in the treatment of cystic fibrosis.

### Background of the invention

Cystic Fibrosis (FC) is the most common life-shortening rare disease in Caucasians [1], mainly characterized by lung disease, the principal cause of morbidity and mortality, including airway obstruction by viscous mucus and inflammation with recurrent bacterial infections and colonization [2]. CF is caused by mutations in the gene coding for CF Transmembrane Conductance Regulator (CFTR), a protein functioning as a chloride channel at the apical membranes of epithelial cells [3]. CFTR is composed by several domains, two membrane-spanning (MSD-1/2), two ATPase (NBD-1/2), and a regulatory (RD) whose phosphorylation regulates channel gating.

To date, the clinical management of CF has failed to address its primary cause, namely the loss-of-function of CFTR. More than 1900 mutations have been identified in the CFTR gene and then categorized in 6 different classes according to their functional impact [4, 5]. Mutation-directed approaches aimed at correcting the CFTR defect have recently emerged [1, 6]. They focus on the identification of molecules that directly target mutant CFTR, either capable of correcting the trafficking of CFTR mutants (correctors) or improving channel function (potentiators). A mutation-directed CFTR repairing therapy with a channel-potentiator (Ivacaftor) is available for ∼5% of CF patients bearing membrane-resident class III mutant (G551D) [7]. In contrast, for the vast majority (70-90%) of CF patients bearing the most common class II F508del mutation, there is no effective treatment [8, 9]. F508delCFTR is retained in the endoplasmic reticulum (ER) and degraded before it reaches the plasma membrane (PM) [10]. Notably that F508delCFTR acquires some degree of function if rescued and stabilized at the PM [11, 12] and restoring about 20-30% of CFTR function is believed to confer at least partial clinical benefit to CF patients [13, 14]. However, when Ivacaftor is combined in vitro with CFTR correctors, the former counteracts the latter by further decreasing F508delCFTR PM stability after rescue [15, 16], thus demonstrating that a novel rationale is needed based on mechanistic rather than mutation-directed approaches.

Alternative strategies, aimed at targeting the cellular environment perturbed by the lack of a functional CFTR instead of directly targeting the mutant CFTR protein, have been recently proposed[17, 18]. In particular autophagy, the major mechanism determining cytoplasmic protein turnover, is blocked due to tissue transglutaminase (TG2)-mediated depletion of the essential autophagy-related protein Beclin 1 (BECN1), leading to secondary accumulation of the autophagic substrate SQSTM1/p62 [19]. Notably that TG2 is a versatile multifunctional protein that acts as a crosslinking enzyme in the presence of high Ca2+ levels, and functions as G-protein or protein disulfide isomerase (PDI) at low Ca2+ concentrations.

Cysteamine has been proposed as a therapeutic agent in view of its ability to restore CFTR function in patients with cystic fibrosis carrying the F508del-CFTR mutation [20, 21]. EP2664326 disclosed a combination of inhibitors of Tissue Transglutaminase (TG2), inhibitors of reactive oxygen species (ROS) such as cystamine or cysteamine and CFTR channel activators for the treatment of CF patients carrying the F508-CFTR mutation. The ability of cysteamine to inhibit the PDI activity of TG2 was also recently confirmed in [22]. As demonstrated before [20], cysteamine is able to restore the 78% of CFTR function at 250µM concentration (see Table 1).

### Description of the invention

It has now been found that cysteamine analogues of formula I are particularly effective in restoring a functional F508delCFTR protein and improving disease phenotype. In addition, the compounds of formula I do not only directly target the F508delCFTR protein, but also other proteins whose malfunction is causal of the low CFTR expression at the cell surface, in particular TG2 and its PDI activity.

The invention accordingly provides compounds of formula I 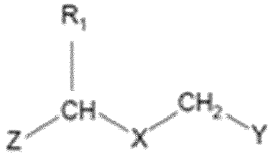 wherein:
- Z is NH₂ or NH₂-NH-, preferably NH₂
- R1 is H, C₁-C₃-alkyl, phenyl optionally substituted by methyl or ethyl, 2-, 3- or 4-pyridyl;
- X is a group of formula -(CH₂)ₙ-, C=O, NH, S or -(CH₂)ₘ-NH-(CH₂)ₒ- or -S -(CH₂)ₚ
- n is zero or an integer from 1 to 4, m is 1 or 2, o is 1 or 2 and p is 1 or 2;
- Y is SH, SeH or SPO₃H (sulfanylphosphonic acid),
for use in the treatment of cystic fibrosis.

In a first group of preferred compounds of formula I , Z is NH₂, R1 is hydrogen, phenyl optionally substituted by methyl or ethyl, 2-, 3- or 4-pyridyl, X is -(CH₂)ₙ-and n is zero when R1 is different from hydrogen or n is 1 to 3 and Y is -SH or SPO₃H.

In a second group of preferred compounds of formula I, Z is NH₂, R1 is hydrogen, X is C=O or -(CH₂)ₙ- wherein n is from 1 to 4 and Y is SH.

In a third group of preferred compounds of formula I, Z is NH₂ or NH₂-NH-, R1 is hydrogen, X is NH or -(CH₂)ₘ-NH-(CH₂)ₒ- wherein m and o are independently 1 or 2 and Y is SH or SPO₃H.

In a fourth group of preferred compounds of formula I, Z is NH₂, X is S or -S -(CH₂)ₚ and Y is -SH.

Preferred compounds of formula I include 3-aminopropane-1-thiol, 4-aminobutane-1-thiol, 5-aminopentane-1-thiol, 6-amino-1-hexanethiol, 2-(3-aminopropyl)aminoethyl phosphorothioate (amifostine). 3-aminopropane-1-thiol is particularly preferred.

The compounds of formula I are either known or may be prepared according to conventional synthetic methods. For the intended therapeutic use, the compounds of the invention will be administered by any suitable route, e.g. by the oral, parenteral or aerosol route. Suitable compositions comprising an effective amount of compounds of formula I and optionally other active ingredients in combination with other pharmaceutically acceptable excipients will be prepared according to known techniques. Examples of said compositions include tablets, capsules, syrups, solutions, suspensions.

The dose of compounds of formula I will be adapted to the specific situation taking into account patient's age, general health conditions, weight, other concomitant therapies and responsiveness to the combined treatment over time. A range of 10 to 1000 mg /dose once or more times a day will be usually acceptable.

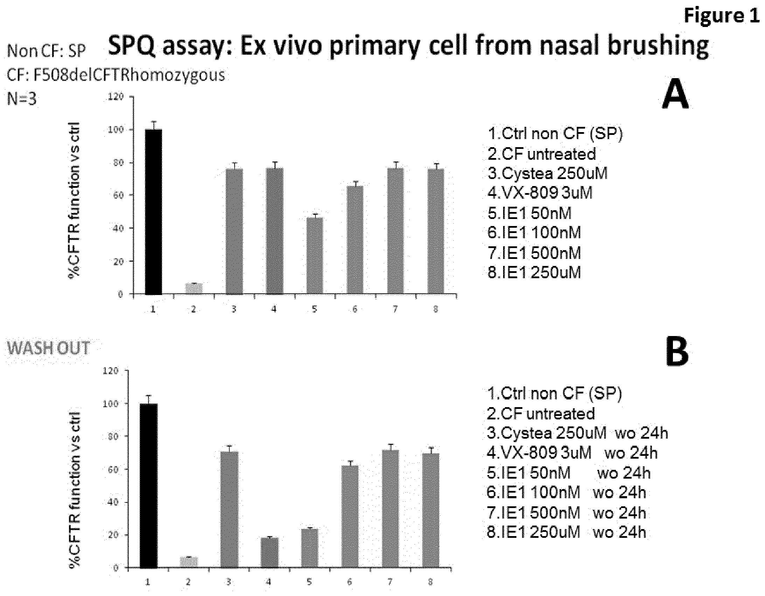

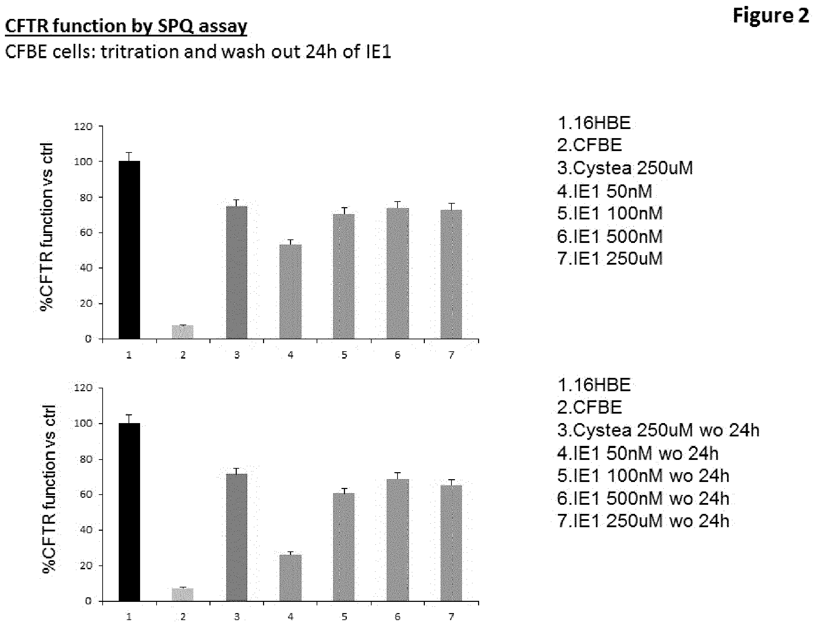

The compounds of formula I have marked effects on CTR function. A representative number of compounds (I) (Table I) were tested through SPQ assay ex vivo in nasal cells collected by nasal brushing from CF patients (Fig. 1A and B) and in F508delCFTR homozygous bronchial epithelial cell lines (CFBE41o-) transfected with F508delCFTR (Fig. 2).

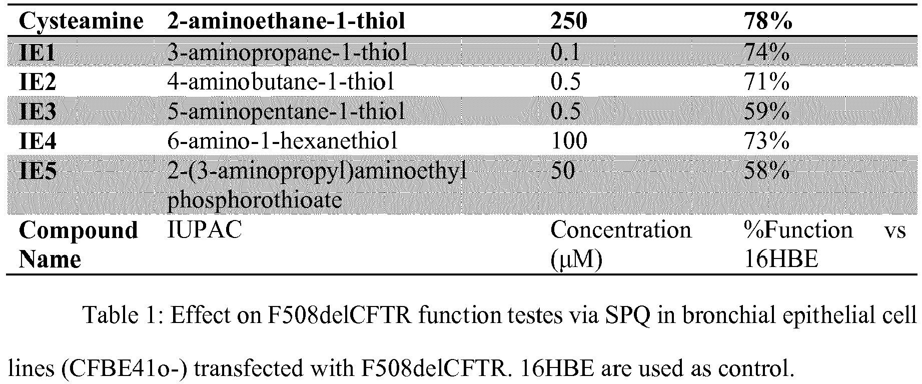

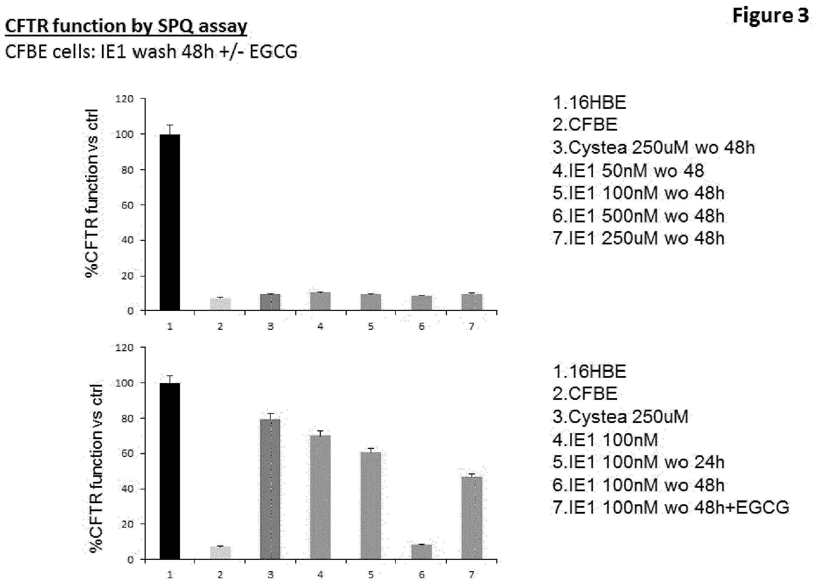

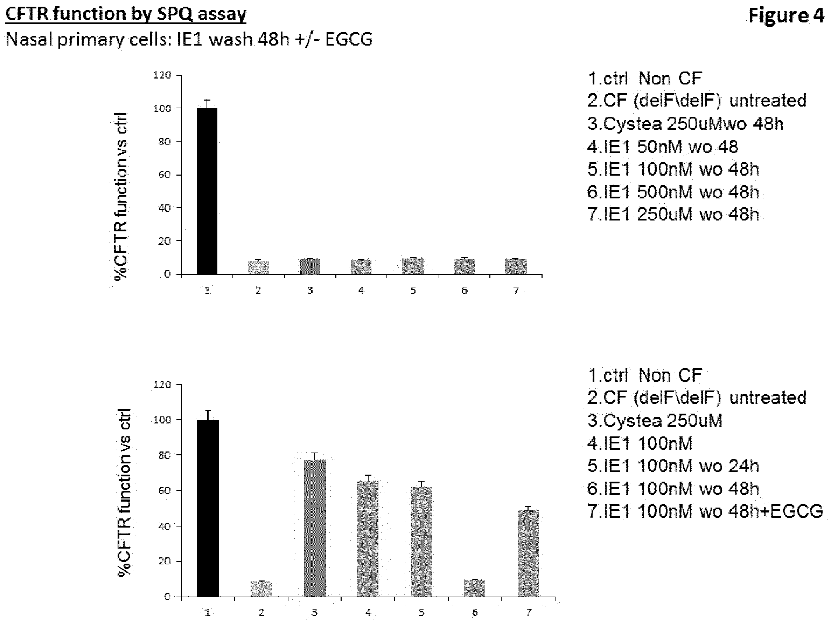

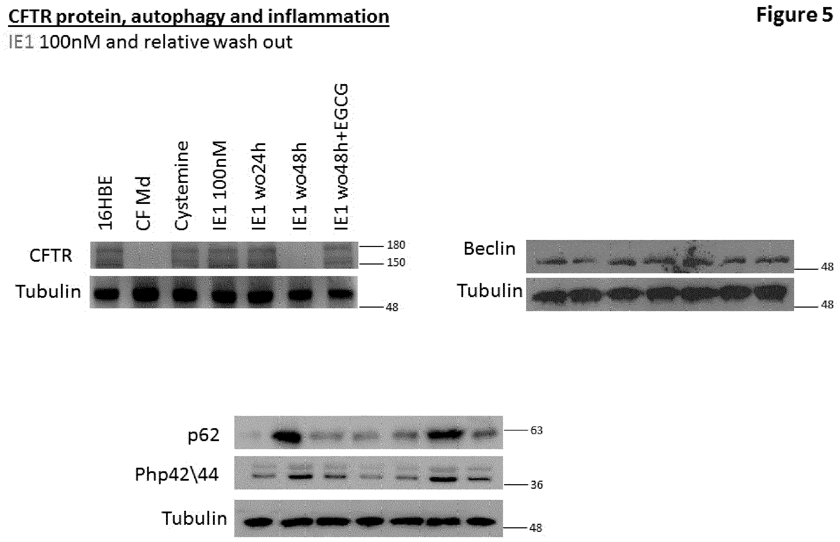

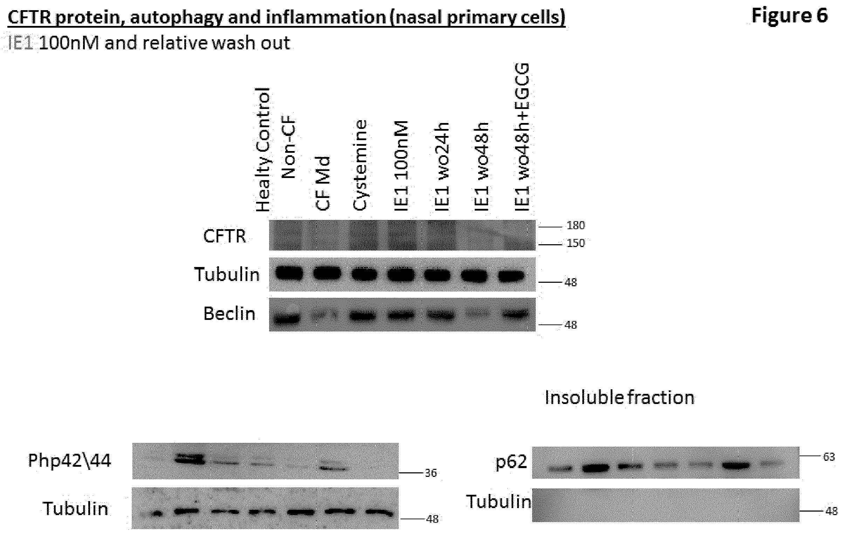

By comparing cysteamine with IE1 it is evident that IE1 is able to restore CFTR function equally to cysteamine, but at a concentration more than 500 fold lower (0.1 µM vs 250 µM). IE1 has been demonstrated more efficacious than VX-809 (3 µM, Fig. 1A) Moreover, as demonstrated for cysteamine, IE1 is able to maintain CFTR function up to 24h after wash out, a result not obtained by the corrector VX-809 (See Fig 1B and Fig 2). To note that IE1 loses its efficacy after 48h after wash out (Fig. 3 and 4), however its effect is restored by adding epigallocatechin gallate (EGCG, Fig. 3 and 4), an effect previously demonstrated for cysteamine [20]. According to the results obtained with IE1 on CFTR function, immunoblot detection of CFTR confirms that a sizeable fraction of the channel resided at the PM after treatment with 0.1 µM IE1 (compared with 250µM cysteamine) and maintained after 24 h of the compound washout. As expected by the function experiments, the amount of PM-resident CFTR protein decreases after 48 h of washout but is completely restored when EGCG is added to the experiment. These results were obtained in both CFBE cells (Fig. 5 and ex vivo cells collected by nasal brushing (Fig. 6). As in the case of cysteamine, the ability of IE1 to inhibit the PDI activity of TG2 was also confirmed (data not shown).

Most of the tested compounds are able to restore more than 50% of CFTR function.

### IE1 effect on authophagy and inflammation

As demonstrated elsewhere, the restoration of Beclin 1-dependent autophagy and the depletion of SQSTM1/p62 by genetic intervention or chemical modulators have positive effects on the CFTR function both in cell and in vivo. As shown in the case of cysteamine, immunoblot analysis shows that IE1 (0.1µM) is able to restore BECN1, to reduce P62 levels with the consequent improvement of CFTR function (see above). This effect disappears after 48h but is restored by EGCG addition. On the other side, the inflammation biomarkers (phospho)p42/44 MAPK decrease in the presence of IE1 (0.1µM), an effect completely lost after 48h but restored in presence of EGCG. These results were obtained by treating both CFBE cells (Fig. 5) and cells collected ex vivo by nasal brushing (Fig. 6).

### Mouse model

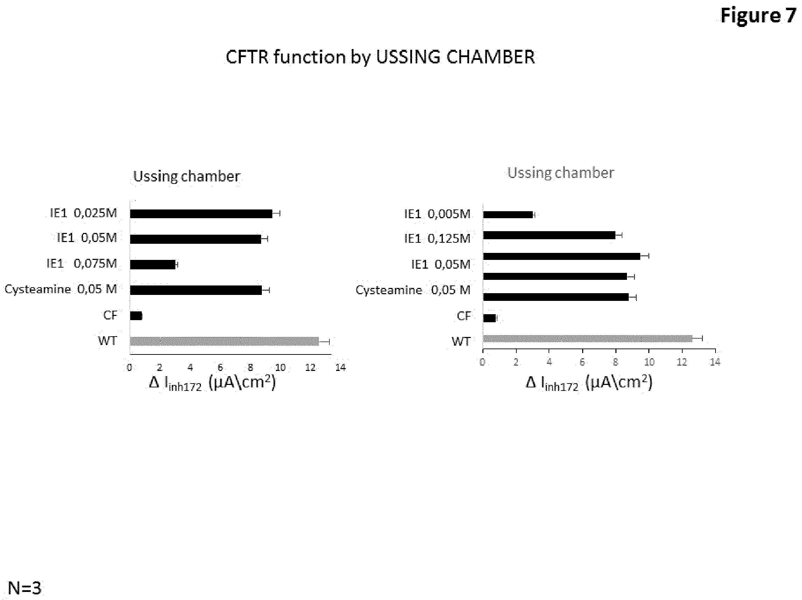

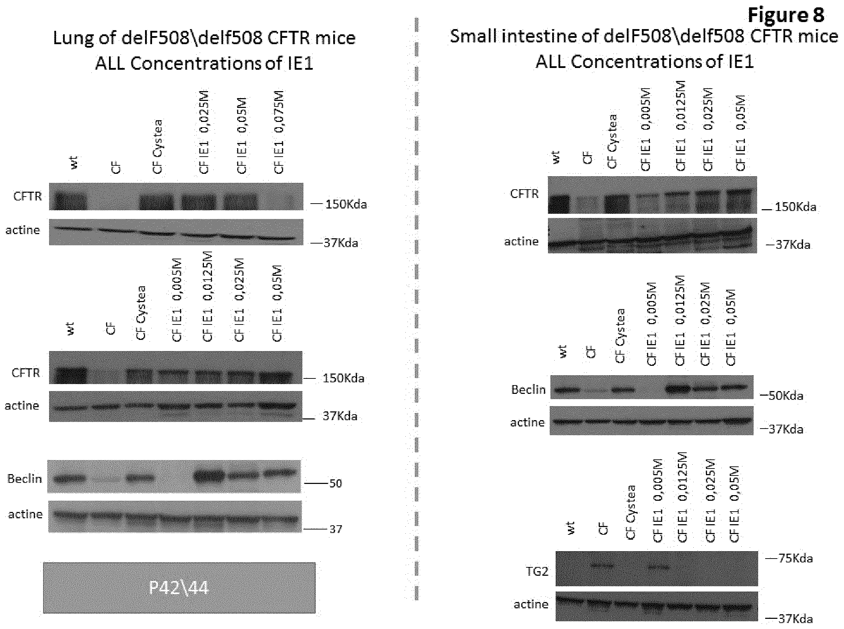

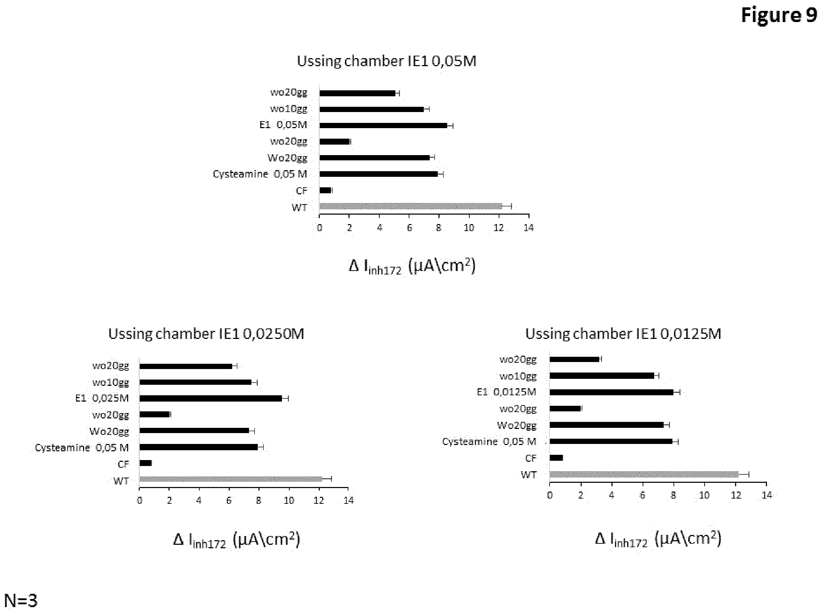

Driven by the positive outcome of the cell lines (immortalized and primary nasal cell) experiments, we next investigated the possibility that IE1 could rescue the CFTR protein and function as well as re-establish the autophagy pathway in CF mice model. To test this hypothesis, we used F508del-Cftr homozygous (CftrF508del\F508del) mice and control mice Wilde type (Cftr WT). CftrF508del mice were administered intraperitoneally (i.p.) for 5 d with cystamine or different concentrations of IE1 (Fig 7). As shown, the Ussing chamber experiments (on small intestinal tissues) revealed that the range concertation of IE1 from 0.05 to 0.00125 are highly effective on rescue the CFTR function. Next, CftrF508del mice were administered intraperitoneally (i.p.) for 5 d with cystamine or 3 different concentration of IE1 (0.05M, 0.025M or 0.0125M) followed by 10 d or 20 d of vehicle only (Fig 9). In this experimental model, the effects of cystamine or IE1 in sustaining the CFTR function highlighted that all of three concentrations are more effective than cystamine to sustain the effect after 20d of wash out. The immunoblots analysis of CFTR protein levels confirm the effect of IE1 on sustaining the protein well beyond 20d of wash out (Fig. 8-9).

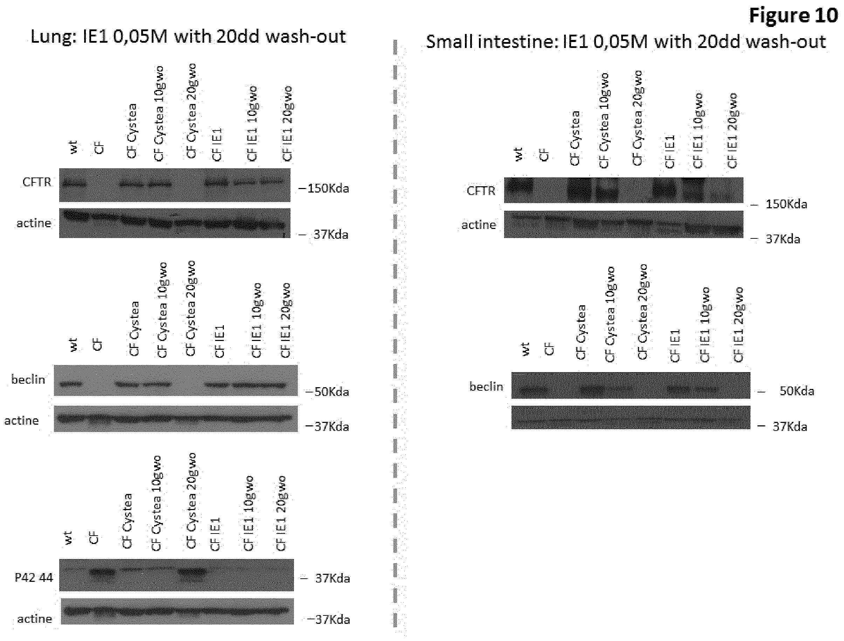

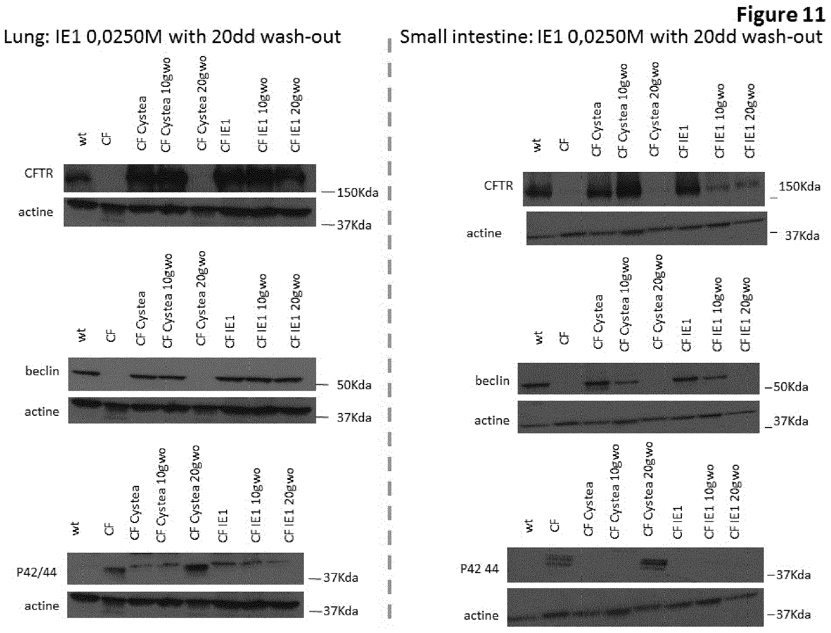

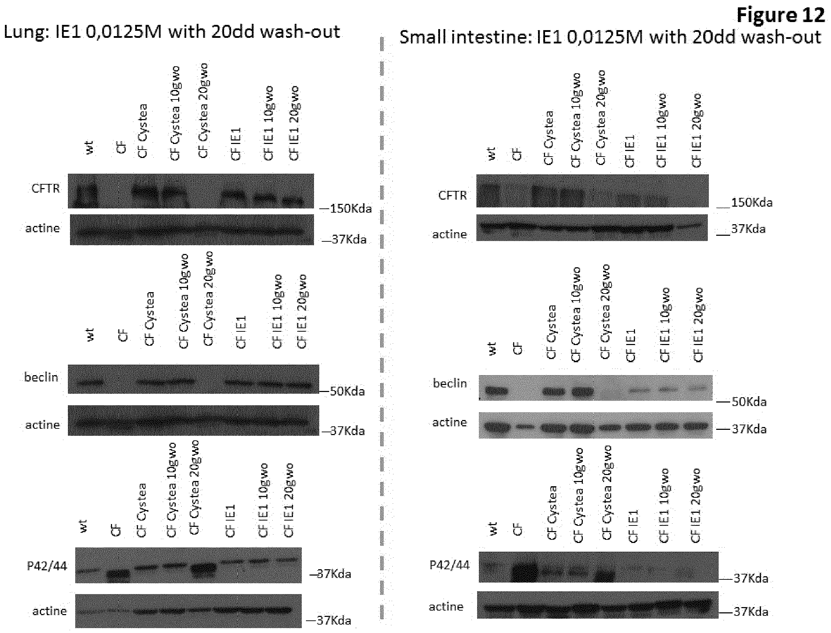

To confirm the effect of IE1 by acting on autophagy and inflammatory pathway, immunoblot analysis of whole homogenates from intestinal and lung tissues showed that IE1 concentrations are able to restore BECN1 and decrease biomarkers (phospho)p42/44 MAPK (Fig. 10-12).

### Methods

**Animal tests.** CF mice homozygous for the ΔF508- CFTR mutation (abbreviated CftrF508del)7,46-48 in the 129/ FVB outbred background (Cftrtm1EUR, F508del, FVB/129) were obtained from Bob Scholte, Erasmus Medical Center Rotterdam, The Netherlands (CF coordinated action program EU FP6 LSHM-CT-2005-018932). These studies and procedures were approved by the local Ethics Committee for Animal Welfare (IACUC N° xxxx). Young adult CF mice and their wild-type (wt) littermates were housed in static isolator cages at the animal care specific pathogen-free facility of Charles River (Calco, Italy). CftrF508del mice were also with daily intraperitoneal injections of cystamine (100 µl of 0.05 M in PBS) (Sigma Aldrich, 30050) or IE1 (100 µl of 0.075-0.05-0.025-0.0125 M, in PBS) for 5 d or with cystamine (5 d) or IE1 different concentrations followed by 10 d or 20d of PBS alone (n = 5 mice per group) (19,20).

**Cell lines experiments.** CFBE41o- and 16HBE14o- (cells (kindly provided by D.C. Gruenert) were cultured in Minimum Essential Medium (MEM) Earles salt (200 mM L-Glutamine, 10% FBS and penicillin/ streptomycin. CFBE41o- and IB3-1 cells were transfected with ΔF508-CFTR at 37°C and incubated for 18 h with cystamine (250 µM) or different concentration of IE1.

### Nasal brushing

Freshly isolated brushed nasal epithelial cells were collected by nasal brushing, as previously described (21,21) from 10 F508del-CFTR homozygous CF patients and from 5 non-CF healthy volunteers recruited from staff at the same time points. Both patient and healthy volunteers gave written informed consent. After nostril washing to remove mucus, cytological brushes (Robimpex, MO11157) were used to scrape the mid part of the inferior turbinate from both nasal nostrils. Brushes with cells were immediately transferred into RPMI 1640 medium (Invitrogen) containing 1% penicillin-streptomycin (Lonza, 17-602E), in 15- ml sterilized tubes. The tubes were incubated at 37°C for 2 h on a thermo shaker, to remove all cell from brushes, the brushes were then removed and the cells centrifuged at 800 x g (2000 rpm) for 20 min. The supernatant fractions were discarded and the cell pellet treated with 150 mL of trypsin-versene (EDTA) solution (Lonza, 17- 161) for 4 min at 37°C to disaggregate possible cell clusters. Trypsin solution was inactivated by adding 3 ml of serum-free Bronchial Epithelial cell Growth Medium BEGM (Clonetics, Lonza, CC3170). After centrifugation at 800 x g (2000 rpm) for 10 min, cells were placed in CELLC T 25 flasks (Sarstedt Ltd, CS300) with 10 ml of BEGM medium. Nonspecific epithelial cells were removed during the daily cell washing and medium changes.

**Immunoblot analysis.** The protein of cell lines or lung or small intestine homogenates was obtained from treated and untreated cells or mice, and the amounts of proteins were determined by a Bio-Rad protein assay to ensure equal protein loading before immunoblot analysis. Fifty micrograms of protein were loaded in each lane. Antibodies against CFTR clone M3A7(Abcam, ab4067) 1:500, SQSTM1, (Sigma Aldrich,108k4767)1:1000, BECN1 (Abcam, ab58878), 1:1000, CFTR clone CF3 (Abcam, ab2784) 1:1000 or anti-CFTR antibody 660 (provided J.R. Riordan through a program of the CFFT, University of North Carolina, Chapel Hill, NC) 1:1000, phospho- ERK1/2 (php42/44, Cell Signaling Technology, #91101) 1:1000. Normalization was performed by probing the membrane with anti-β-actin (Cell Signaling, #4970) 1:1000.

### References

[1] K. DE BOECK ET AL., J CYST FIBROS, 13 (2014) 403-409.
[2] F. RATJEN ET AL., CYSTIC FIBROSIS, LANCET, 361 (2003) 681-689.
[3] J.R. RIORDAN ET AL., SCIENCE, 245 (1989) 1066-1073.
[4] M.D. AMARAL ET AL., CURR PHARM DES, 19 (2013) 3497-3508.
[5] M.D. AMARAL ET AL., TRENDS PHARMACOL SCI, 28 (2007) 334-341.
[6] M.D. AMARAL, J INTERN MED, 277 (2015) 155-166.
[7] B.W. RAMSEY, ET AL., N ENGL J MED, 365 (2011) 1663-1672.
[8] A.M. JONES ET AL., THORAX, 70 (2015) 615-616.
[9] M.P. BOYLE ET AL., LANCET RESPIR MED, 2 (2014) 527-538.
[10] C.M. FARINHA ET AL., FEBS J, 280 (2013) 4396-4406.
[11] G.L. LUKACS ET AL., J BIOL CHEM, 268 (1993) 21592-21598.
[12] G.L. LUKACS ET AL., TRENDS MOL MED, 18 (2012) 81-91.
[13] E. KEREM, CURR OPIN PULM MED, 10 (2004) 547-552.
[14] A.S. RAMALHO ET AL., AM J RESPIR CELL MOL BIOL, 27 (2002) 619-627.
[15] D.M. CHOLON ET AL., SCI TRANSL MED, 6 (2014) 246RA296.
[16] G. VEIT, R.G. ET AL., SCI TRANSL MED, 6 (2014) 246RA297.
[17] W.E. BALCH, D.M. ROTH, D.M. HUTT, EMERGENT PROPERTIES OF PROTEOSTASIS IN MANAGING CYSTIC FIBROSIS, COLD SPRING HARB PERSPECT BIOL, 3 (2011).
[18] S. PANKOWET AL., NATURE, 528 (2015) 510-516.
[19] A. LUCIANI ET AL., NAT CELL BIOL, 12 (2010) 863-875.
[20] D. DE STEFANO ET AL., AUTOPHAGY, 10 (2014) 2053-2074.
[21] A. TOSCO ET AL., CELL DEATH DIFFER, 23 (2016) 1380-1393.
[22] F. ROSSIN ET AL., EMBO REP, (2018).

## Claims

1. Compounds of formula I 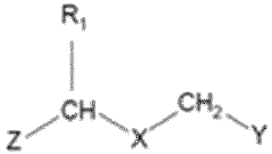 wherein:
- Z is NH₂ or NH₂-NH-, preferably NH₂
- R1 is H, C₁-C₃-alkyl, phenyl optionally substituted by methyl or ethyl, 2-, 3- or 4-pyridyl;
- X is a group of formula -(CH₂)ₙ-, C=O, NH, S or -(CH₂)ₘ-NH-(CH₂)ₒ- or -S -(CH₂)ₚ
- n is zero or an integer from 1 to 4, m is 1 or 2, o is 1 or 2 and p is 1 or 2;
- Y is SH, SeH or SPO₃H,
for use in the treatment of cystic fibrosis.

2. Compounds for use according to claim 1 wherein Z is NH₂, R1 is hydrogen, phenyl optionally substituted by methyl or ethyl, 2-, 3- or 4-pyridyl, X is -(CH₂)ₙ-and n is zero when R1 is different from hydrogen or n is 1 to 3 and Y is -SH or SPO₃H.

3. Compounds for use according to claim 1 wherein Z is NH₂, R1 is hydrogen, X is C=O or -(CH₂)ₙ- wherein n is from 1 to 4 and Y is SH.

4. Compounds for use according to claim 1 wherein Z is NH₂ or NH₂-NH-, R1 is hydrogen, X is NH or -(CH₂)ₘ-NH-(CH₂)ₒ- wherein m and o are independently 1 or 2 and Y is SH or SPO₃H.

5. Compounds for use according to claim 1 wherein Z is NH₂, X is S or -S -(CH₂)ₚ and Y is -SH.

6. Compounds for use according to claim 1 selected from 3-aminopropane-1-thiol, 4-amino butane-1 -thiol, 5-aminopentane-1-thiol, 6-amino-1 -hexanethiol, 2-(3-aminopropyl)aminoethyl phosphorothioate.

7. A compound for use according to claim 1 which is 3-aminopropane-1-thiol.
